Europäisches Patentamt

European Patent Office

Office européen des brevets

(11) Veröffentlichungsnummer: **0 053 392**
**A2**

(12) **EUROPÄISCHE PATENTANMELDUNG**

(21) Anmeldenummer: **81110056.9**

(22) Anmeldetag: **02.12.81**

(51) Int. Cl.³: **A 61 M 1/03**

(30) Priorität: **03.12.80 DE 3045495**

(43) Veröffentlichungstag der Anmeldung: **09.06.82**
**Patentblatt 82/23**

(84) Benannte Vertragsstaaten: **FR GB IT**

(71) Anmelder: **SECON Gesellschaft für Separations- und Concentrationstechnick mbH, Wagenstieg 5, D-3400 Göttingen (DE)**

(72) Erfinder: **Weickhardt, Ludwig, Hasenwinkel 8, D-3406 Bovenden 1 (DE)**

(74) Vertreter: **Patentanwälte Dipl.-Ing. Rudolf Bibrach Dipl.-Ing. Elmar Rehberg, Pütterweg 6, D-3400 Göttingen (DE)**

(54) Dialysator aus aufeinandergestapelten semipermeablen Schlauchabschnitten.

(57) Bei einem Dialysator mit einem Gehäuse und daran angeordneten Zu- und Ableitungen für Blut und Dialysat sind in dem Gehäuse aufeinandergestapelte semipermeable Schlauchabschnitte angeordnet, die jeweils in ihrem Innern eine Membranhalterung aus ungewebtem Kunststoffmaterial sich kreuzender Fäden aufweisen. In den Schlauchabschnitten ist der Strömungspfad für das Dialysat und zwischen den Schlauchabschnitten der dazu kreuzweise geführte Strömungspfad für das Blut gebildet. Um einen Dialysator zu zeigen, der in Verbindung mit gut entgasenden wie auch mit schlecht entgasenden Dialysiermaschinen etwa den gleichen Druckabfall aufweist und bei minimaler Blutfilmdicke optimale Eigenschaften zeigt, ist die Membranhalterung in Fliessrichtung des Dialysats durch den Einsatz vergleichsweise dicker Kettfäden auf gegenseitigem Abstand eine Vielzahl hindernisfreier Dialysatwege gebildet. Die Membranhalterung besitzt in Fliessrichtung des Blutes durch den Einsatz demgegenüber vergleichsweise dünner Schussfäden auf gegenseitigem Abstand eine Vielzahl hindernisfreier Blutwege geringer Filmdicke.

Dialysator aus aufeinandergestapelten semipermeablen Schlauchabschnitten

Die Erfindung bezieht sich auf einen Dialysator mit einem Gehäuse und daran angeordneten Zu- und Ableitungen für Blut und
Dialysat und mit in dem Gehäuse angeordneten, aufeinandergestapelten semipermeablen Schlauchabschnitten, die jeweils in
ihrem Innern eine Membranhalterung aus ungewebtem Kunststoffmaterial sich kreuzender Fäden aufweisen, die in den Schlauchabschnitten den Strömungspfad für das Dialysat und zwischen
den Schlauchabschnitten den dazu kreuzweise geführten Strömungspfad für das Blut bildet.

Der artige Dialysatoren sind aus der DE-OS 20 24 635 bekannt.
Die Membranhalterung, die in jedem Schlauchabschnitt angeordnet wird, besteht aus möglichst dünnem, ungewebten Material,
also aus Kunststoffäden, die kreuzweise übereinandergelegt
sind und durch Wärmeeinwirkung aneinander stabilisiert sind.
Fäden besitzen, gleichgültig in welcher Richtung sie eingesetzt
werden, etwa gleichen Durchmesser. Sofern das Dialysat im
Innern der Schlauchabschnitte und an den Membranhalterungen
vorbeigeführt wird, stellt die Membranhalterung ein beachtliches Hindernis dar, an dem sich insbesondere in Verbindung
mit schlecht entgasenden Maschinen Gasblasen abscheiden, die
zu einer Erhöhung des Widerstandes und damit zu einem Druckabfall auf der Dialysatseite führen. In Verbindung mit gut
entgasenden Maschinen tritt ein solcher Druckabfall auf der
Dialysatseite kaum auf. Somit ergibt sich der Nachteil, daß
der Dialysator scheinbar unterschiedliche Eigenschaften besitzt, je nachdem, mit welcher Maschine er in Zusammenhang
eingesetzt wird. Dies ist für die Bedienung einer Maschine
und eines Dialysators störend, ganz abgesehen davon, daß in
Verbindung mit einer schlecht entgasenden Maschine unter Umständen die vorgesehene Entwässerung des Patienten durch
Ultrafiltration nicht erreicht wird.

Der gleiche Nachteil wird auch bei dem Kreuzriefendialysator nach Hoeltzenbein beobachtet. Dies ist nicht weiter verwunderlich, da ja die parallelen Riefen ganz ähnliche Eigenschaften hervorrufen, wie die Verwendung einer ungewebten Membranhalterung unter Verwendung von parallelen Fäden. Dies kann dazu führen, daß bei entsprechend großer Gasabscheidung im Kreuzriefendialysator ein solcher Druckabfall eintritt, daß die dabei eingesetzte Maschine infolge dort vorgesehener Sicherheitsvorrichtungen abschaltet und somit den Dialysevorgang unterbricht.

Jeder Dialysator, gleich ob Spule, Kapillare oder Platten, wirkt im Dialysatteil wie ein Gasabscheider. Je kleiner die Dialysatwege, desto stärker ist die Gasabscheidung. Große Gasblasen werden z. B. von plattenförmigen Dialysatoren erzeugt. Wenn diese Blasen in den Membranhalterungen hängenbleiben, so steht die durch die Blasen nicht überströmte Membranfläche für die Diffusion nicht mehr zur Verfügung. Bei kleinen oder mittelgroßen Blasen werden die Diffusionswege zu lang. In beiden Fällen wird die Gesamtclearance verringert.

Bei dem aus der DE-OS 27 54 220 bekannten Dialysator wird als Membranhalterung ein spezielles Gewebe verwandt, dessen Zwischenräume einer Seitenlänge von 0,3 mm haben. Dadurch wird unter anderem erreicht, daß die sonst üblichen großen Gasblasen in kleinste Blasen mit einem maximalen Radius von 0, 15 mm verteilt werden. Diese Blasen sitzen in den Gewebeöffnungen. Diese Gasblasen werden im Betriebszustand vom Dialysat umströmt. Die Diffusionsstrecke beträgt demnach nur maximal 0, 15 mm. Daher wird die Diffusion auch bei gasgefülltem Dialysatteil aufrechterhalten. Diese Wirkungsweise kann man belegen, indem man die Clearancen einer nicht entgasenden Dialysier-Maschine mit einer gut entgasenden Dialysier-Maschine vergleicht. Die aufgetretene Differenz ist auf
die niedrigere Dialysatmenge pro Minute bei der nicht entgasenden Maschine zurückzuführen. Gasgefüllte Dialysatteile haben also bei diesem bekannten Dialysator keinen Einfluß auf
die Clearance. Bei der Messung und Einstellung der Ultrafiltration sind bei der Anwendung schlecht entgasender Maschinen
jedoch einige meßtechnische Dinge zu beachten, die die Anwendung erschweren. Die Gasblasen wirken als Strömungswiderstand
auf das Dialysat. Bei schlechter Dialysatentgasung füllen die
Blasen innerhalb von 15 bis 30 Minuten die freien Gewebeöffnungen aus. Dieser Widerstand kann bis zu ca. 160 mm Hg ansteigen. Nach 30 Minuten ist ein stationärer Zustand erreicht,
d. h. sämtliche freien Gewebeöffnungen sind gasgefüllt. Neuabgeschiedenes Gas wird nun vollständig abgeführt.

Auch bei sehr gut entgasenden Maschinen sind speziell bei hohem Transmembrandruck, also hoher Druckdifferenz an der
Membran, vereinzelte Gasblasen zu beobachten. Hier kann es
sich infolge der hohen Durchlässigkeit der Membran um
diffundiertes $CO_2$ aus dem Blut oder aber um "Luftziehen" in
der Dialysatkupplung handeln. Eine Erhöhung des Strömungswiderstandes wird hierbei nicht in dieser Größenordnung auftreten. Der erhöhte Strömungswiderstand bei schlecht entgasenden Maschinen wirkt sich auf die Messung und Einstellung
der Ultrafiltrationsrate des Dialysators aus, und zwar in Abhängigkeit von der Art und Weise, wie und wo der Transmembrandruck gemessen wird. Die in der Praxis übliche Ermittlung
der Ultrafiltrationsrate kann solche Situationen nicht erfassen, da sie weden der Ort der Druckmessung, noch den Strömungswiderstand berücksichtigt. Da beispielsweise nur die ausgangsseitig am Blut und am Dialysat auftretenden Drücke be-

0053392

rücksichtigt werden, ergibt sich ein scheinbarer Transmembrandruck, der je nach den Verhältnissen mehr oder weniger von dem realen Transmembrandruck abweicht. Zu einer exakteren Ermittlung der Ultrafiltrationsrate muß der eingangsseitige und der ausgangsseitige Druck auf der Blutseite und auf der Dialysatseite gemessen werden. Durch Mittelwertbildung ergibt sich ein Transmembrandruck, der den tatsächlichen Verhältnissen sehr nahekommt. Da aber bei den meisten Dialysiermaschinen eine eingangsseitige und eine ausgangsseitige Druckmessung am Blutweg und am Dialysatweg nicht vorgesehen ist, sondern man sich in der Regel mit der ausgangsseitigen Messung begnügt, kann die Ultrafiltrationsrate nur anhand des scheinbaren Transmembrandruckes ermittelt werden, so daß sie von einer großen Unsicherheit behaftet ist, die davon abhängt, wie gut bzw. wie schlecht die zugehörige Dialysiermaschine entgast. Beispielsweise bei Verwendung einer schlecht entgasenden Maschine und bei niedriger Entwässerung des Patienten wird sich eine Druckeinstellung empfehlen, die entsprechend niedrig ist. Dabei kann das Verhältnis des scheinbaren Transmembrandruckes zu dem realen Transmembrandruck sich wie 2 : 3 verhalten.

Der Erfindung liegt daher die Aufgabe zugrunde, einen Dialysator der eingangs beschriebenen Art meit einer Membranunterstützung für dünne Dialysatfilme zu schaffen, die auf der Dialysatseite einen möglichst geringen Druckabfall aufweist, wenn aus dem Dialysat Gas abgeschieden wird. Anders gesagt, soll der Dialysator so ausgebildet sein, daß er unabhängig davon, ob er im Zusammenhang mit einer gut entgasenden oder einer schlecht entgasenden Dialysiermaschine eingesetzt wird, der Druckabfall sich in relativ engen Grenzen verändert, also so klein ist, daß er für die Ultrafiltrationsrate nicht unzumutbare Abweichungen zeigt.

Erfindungsgemäß wird dies dadurch erreicht, daß die Membranhalterung in Fließrichtung des Dialysats durch den Einsatz vergleichsweise dicker Kettfäden auf Abstand eine Vielzahl hindernisfreier Dialysatwege aufweist, und daß die Membranhalterung in Fließrichtung des Blutes durch den Einsatz vergleichsweise dünner Schußfäden auf Abstand eine Vielzahl dünner Strömungspfade für das Blut besitzt, und daß die Schußfäden auf den Kettfäden stabilisiert sind. Die Erfindung geht davon aus, die unterschiedlichen Eigenschaften des Blutes einerseits und des Dialysats andererseits auch hinsichtlich der Ausgestaltung der Membranhalterung zu berücksichtigen und dem Dialysat einen weitgehend hindernisfreien Weg zur Verfügung zu stellen, damit sich Gasblasen kaum festsetzen können und somit ein Druckabfall in Verbindung mit schlecht entgasenden Maschinen kaum eintritt. Im Gegensatz zur Verwendung eines Kreuzriefengitters oder eines entsprechenden Gewebes als Membranhalterung, bei dem jedem Dialysatpfad eine große Vielzahl von einzelnen Kammern, die mit je einer Gasblase gefüllt sein können, gebildet wird, wird hier jeweils in einem Dialysatweg bestensfalls nur noch eine Gasblase angeordnet. Infolge dessen sind die Kapillarkräfte, die im Dialysatweg bei der erfindungsgemäßen Ausbildung zu überwinden sind, sehr viel geringer. Andererseits ist es erforderlich, die Blutfilmdicke möglichst zu erniedrigen, also dem Blut eine Vielzahl dünner Strömungspfade zur Verfügung zu stellen, die hinsichtlich ihrer Größenordnung auch ausreichend stabil über eine längere Zeit sind. Bei einer solchen erfindungsgemäßen Ausbildung der Membranhalterung wird der Vorteil erreicht, daß der Dialysator selbst sehr kleine Abmessungen und ein geringes Gewicht besitzen kann, andererseits aber eine relativ große Membranfläche auf kleinstem Raum untergebracht ist. Dies ist wünschenswert und in vieler Hinsicht vorteilhaft, beispielsweise resultiert hieraus ein geringes Blutfüllvolumen. Dementsprechend ergibt sich wiede-

0053392

rum eine problemlose Blutrückgabe mit geringen Mengen Spülflüssigkeit am Ende der Dialyse. Vor allen Dingen aber ändert sich der Transmembrandruck nicht sehr - unabhängig ob
der Dialysator in Verbindung mit einer schlecht oder gut entgasenden Dialysier-Mschine eingesetzt wird. Damit kann die
Ultrafiltration linear über einen großen Zeitraum und Bereich gesteuert werden. Es versteht sich, daß die hinsichtlich ihrer Kett- und Schußfäden unterschiedlich ausgebildete Membranhalterung dann auch in der vorgegebenen Ausrichtung innerhalb der semipermeablen Schlauchabschnitte angeordnet wird.

Die Kett- und Schußfäden können mit solchen geometrischen
Abmessungen vorgesehen sein, daß die Schlauchabschnitte möglichst wenig durchhängen und somit ein Strömungspfad von
einer mittleren Dicke von etwa 50 $\mu$ gebildet wird. Damit
wird ein dünner Blutfilm an der Membranfläche zur Einwirkung gebracht, so daß die Diffusionswege vergleichsweise
kurz sind.

Die Kettfäden bestimmen im wesentlichen die Stabilität der
Membranhalterung, wobei die Stabilisierung der Schußfäden
auf den Kettfäden durch eine Verschweißung und/oder eine
Schlingenbildung unter Einsatz eines dünnen Schlingfadens
bewirkt ist. Dieser Schlingfaden ist so dünn, daß er die
hindernisfreien Dialysatwege nicht behindert und auch die
Blutfilmdicke nicht beeinträchtigt. Die die hindernisfreien
Dialysatwege bildenden Kettfäden weisen einen Durchmesser
auf, der einerseits für eine Abfuhr abgeschiedenen Gases
ausreichend groß und andererseits für die Bildung dünner
Dialysatfilme hinreichend klein ist. Unter Beachtung der
physikalischen Grenzwerte ist eine Optimierung dieser beiden
gegenläufigen Forderungen zu erreichen. Dabei können die Kettfäden einen Durchmesser zwischen 0, 15 bis 0, 3 mm -
vorzugsweise 0, 2 mm -, einen gegenseitigen Abstand zwischen
0, 5 mm bis 1, 0 mm - vorzugsweise 0, 6 mm -, die Schußfäden
einen Durchmesser von 0, 08 mm bis 0, 15 mm - vorzugsweise
0, 12 mm - und einen gegenseitigen Abstand von 1, 0 bis
2, 0 mm - vorzugsweise 1, 2 bis 1, 6 mm - aufweisen. Die angegebenen Bereiche sind günstig für die Verhinderung der
Änderung des Transmembrandruckes in allzu großen Bereichen.
Wird beispielsweise der Kettfaden zu dick gewählt, also
beispielsweise dicker als 0, 3 mm, dann führt dies zu einer
Einschränkung der Dialysierleistung. Wird der Abstand der
Schußfäden zu groß gewählt, dann hängt die Membran zwischen
diesen zu sehr durch, so daß sich die mittlere Blutfilmdicke
in nachteiliger Weise erhöht.

Bei Stabilisierung des Schußfadens auf dem Kettfaden durch
einen Schlingfaden kann dieser einen Durchmesser von etwa
0, 08 mm aufweisen, so daß er gegenüber den Kett- und Schußfäden nicht störend ins Gewicht fällt, andererseits aber
eine gute technische Realisierungsmöglichkeit für die Stabilisierung der Schußfäden auf den Kettfäden bildet. Der Kettfaden kann aus Polyäthylen, der Schußfaden aus Polyester oder
Polyacryl und der Schlingfaden aus Polyamid bestehen.

Es ist auch möglich, nicht nur jeweils einen Schußfaden mit
Abstand zum Nachbarschußfaden auf den Kettfäden anzuordnen,
sondern auf einzelnen abständig angeordneten Kettfäden je
zwei Schußfäden unmittelbar nebeneinander und diese Schußfadenpaare dann wieder abständig zueinander zu stabilisieren.
Diese herstellungsbedingte Ausführung hat sich als sehr sinnvoll erwiesen.

Bei Einsatz eines Schlingfadens können vorteilhaft mindestens zwei verschiedene Arten von Membranhalterungen abwechselnd im Stapel angeordnet sein, die sich dadurch unterscheiden, daß sie jeweils verschiedenen Abstand benachbarter Schußfäden aufweisen. Auf diese Weise wird das geordnete Einrutschen von Membranhalterungen in Nachbarmembranhalterungen und damit die unkontrollierte Änderung der Stapelhöhe vermieden. Wenn die beiden Abstände auf einem gemeinsamen Teilungsraster aufgebaut sind und jeweils ein unterschiedliches ganzzeiliges Vielfaches des Teilungsraster ausmachen, erhält man in regelmäßig wiederkehrenden Abständen eine präzise Abstützung zweier benachbarter Membranhalterungen durch die anliegenden Membrane hindurch, und zwar gleichgültig, in welcher relativen Verschiebungslage sich die beiden Membranhalterungen gegenseitig befinden. Dies aufaddiert über die gesamte Stapelhöhe bringt eine verläßliche reproduzierbare Abstützung der einzelnen Membranhalterungen aneinander, eine reproduzierbare Stapelhöhe, reproduzierbare dünne Blutfilmwege und eine gute Aufteilung des Blutes in die einzelnen Blutfilmwege. Auch hinsichtlich der Anordnung der Abdichtung des Membranstapels ergeben sich Vorteile bei der Herstellung.

Die Erfindung wird anhand einiger Ausführungsbeispiele weiter erläutert und beschrieben. Es zeigen:

Fig. 1    einen Ausschnitt aus einer perspektivischen Darstellung der Membranhalterung in einer ersten Ausführungsform,

Fig. 2    einen Ausschnitt aus einer perspektivischen Darstellung der Membranhalterung in einer zweiten Ausführungsform,

Fig. 3    einen Querschnitt durch einen Teil des Membranstapels einer dritten Ausführungsform gemäß der
         Linie III-III ind Fig. 4    und

Fig. 4    einen Schnitt durch die Ausführungsform gemäß

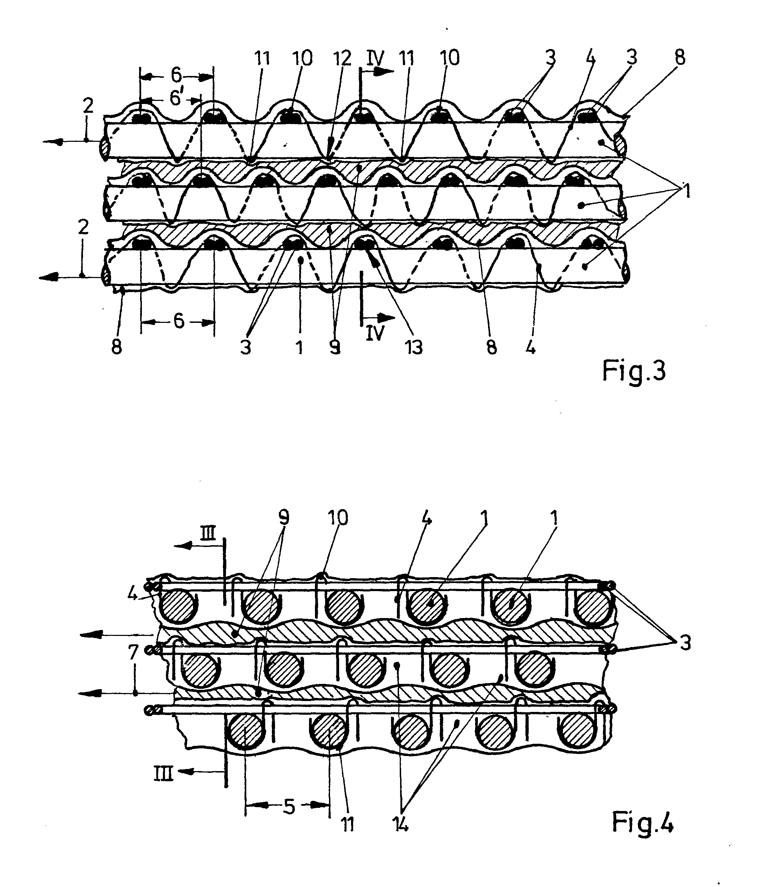

         Fig. 3 nach der Linie IV-IV.

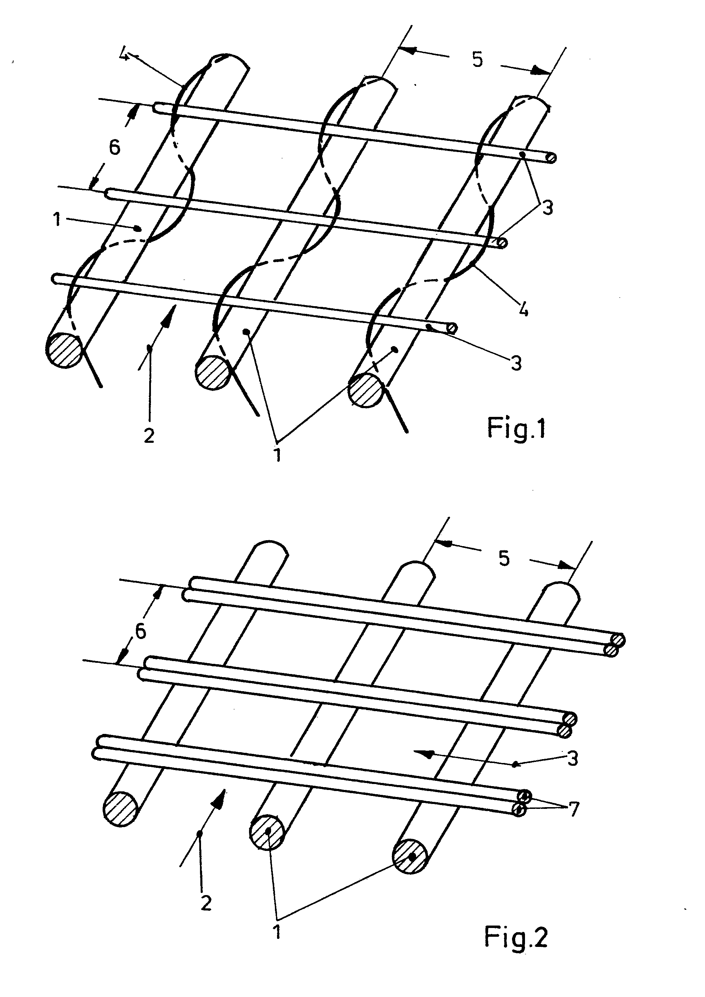

Der Aufbau des Dialysators entspricht im wesentlichen demjenigen, wie er in der DE-OS 27 54 220 gezeigt ist und damit
als bekannt vorausgesetzt wird. Dabei sind eine Mehrzahl
semipermeabler Schlauchabschnitte übereinandergestapelt, wobei jeweils im Innern jedes Schlauchabschnittes eine Membranhalterung aus ungewebtem Kunststoffmaterial sich kreuzender
Fäden vorgesehen ist. Eine solche Membranhalterung zeigt im
Ausschnitt und in stark vergrößernder Darstellung Fig. 1. Die
Membranhalterung besteht aus vergleichsweise dicken Kettfäden
1, die mit Abstand zueinander angeordnet sind und sich in
Fließrichtung 2 des Dialysates erstrecken. In dieser Richtung
erstreckt sich auch die Achse jedes Schlauchabschnittes. Quer
dazu sind vergleichsweise dünnere Schußfäden 3 gleichabständig
auf die Kettfäden 1 aufgelegt und mit Hilfe je eines Schlingfadens 4 stabilisiert. Wie ersichtlich, besitzt der Schlingfaden 4 einen sehr kleinen Durchmesser. Die Kettfäden 1 bestehen aus Polyäthylen und besitzen beispielsweise einen Durchmesser von 0, 2 mm und einen gegenseitigen Abstand 5 von
0, 6 mm. Die Schußfäden 3 bestehen aus Polyester und besitzen
einen Durchmesser von 0, 12 mm und einen gegenseitigen Abstand
6 von 1, 5 mm. Der Schlingfaden 4 besteht aus Polyamid und besitzt einen Durchmesser von 0, 08 mm. Die Kettfäden 1 bestimmen im wesentlichen die Stabilität der Membranhalterung. Die
auf ihnen stabilisierten Schußfäden 3 dienen im wesentlichen
der Membranauflagerung auf der einen Seite, während andererseits die Membran auf der Unterseite der Kettfäden 1 aufliegt.

Fig. 2 zeigt eine ähnliche Ausbildung, bei der jedoch jeweils zwei Schußfäden 3 unmittelbar nebeneinanderliegend angeordnet sind und der Abstand 6 zwischen zwei benachbarten Schußfadenpaaren eingehalten wird. Auf die Verwendung eines Schlingfadens 4 zur Stabilisierung ist hier verzichtet. Statt dessen sind die Kunststoffäden durch eine thermische Behandlung aneinander fixiert bzw. stabilisiert. Auch hier erstrecken sich die Kettfäden 1 in Fließrichtung 2 des Dialysats, während die Schußfäden 3 in Fließrichtung 7 des Blutes angeordnet sind.

Die Fig. 3 und 4 zeigen eine Ausführungsform in Querschnittsdarstellung, die sich durch die Kombination der Ausführungsformen gemäß Fig. 1 und 2 ergibt, nämlich die Verwendung von jeweils zwei Schußfäden 3 unmittelbar benachbart nebeneinander und durch die Stabilisierung mit Hilfe eines Schlingfadens 4. Es sind jeweils drei Lagen übereinander dargestellt, so wie sich diese in einem Stapel ergeben. Fig. 3 zeigt einen Schnitt durch die Schußfäden 3, so daß die Kettfäden 1 in der Zeichenebene waagerecht verlaufen. Jede der drei Membranhalterungen ist von einer schlauchförmigen Membran 8 umgeben, so daß zwischen zwei Membranen ein Strömungspfad 9 für den Blutfilm gemäß der Fließrichtung 7 gebildet ist. Das Dialysat fließt im Bereich der Membranhalterung senkrecht zur Zeichenebene. Fig. 3 veranschaulicht die geringe mittlere Dicke der Strömungspfade 9 für das Blut, die ungefähr in der Größenordnung von 50 μ liegt.

In dem Stapel gemäß Fig. 3 sind zwei verschiedene Arten von Membranhalterungen eingesetzt, die jeweils abwechselnd angeordnet werden. Die oberste und unterste Membranhalterung ist von gleicher Art, während die mittlere Membranhalterung davon abweicht. Diese Abweichung besteht darin, daß die Schußfadenpaare 3 voneinander einen gleichen vergleichsweise größer gewählten Abstand 6 voneinander aufweisen, während die mittlere Lage einen vergleichsweise kleineren Abstand 6´ besitzt. Der Abstand 6 möge beispielsweise 1, 5 mm und der Abstand 6´ beispielsweise 1, 25 mm betragen. Der Schlingfaden 4 steht bei seiner Umschlingung der Schußfäden 3 über diesen mit seinem Teil 10 nach oben vor, während er an der Umschlingungsstelle des Schußfadens 1 mit seinem Teil 11 jeweils nach unten vorsteht. Durch die unterschiedliche Ausbildung der Abstände 6 und 6´ und die alternierende Anordnung dieser beiden Arten von Membranhalterungen verschiebt sich die Überdeckungsstelle der Teile 10 und 11 des Schlingfadens 4 benachbarter Membranen jeweils gegeneinander. In vergleichsweise größeren Abständen wird erreicht, daß sich ein Teil 11 des Schlingfadens auf einem Teil 10 eines benachbarten Schlingfadens abstützt, wie dies an der Stelle 12 der Fall ist. Diese Stellen 12 wiederholen sich in regelmässigen Abständen quer durch den Stapel, also entlang der Richtung des Pfeiles 7. Es werden auf diese Weise Abstützstellen 12 gebildet, die einen reproduzierbaren Abstand zwischen benachbarten Membranen und damit auch eine reproduzierbare Stapelhöhe der Membranen in dem Dialysator garantieren, und zwar unabhängig davon, ob und wie weit die Membranen in ihrer zufälligen Lage gegeneinander verschoben sind (in Richtung der Pfeile 7). Zwischen der mittleren Lage und der unteren Lage ist eine weitere Abstützstelle 13 dargestellt. Denkt man sich eine Membranhalterung in Richtung der Pfeile 7 relativ verschoben, so ändert lediglich die Abstützstelle 12 bzw. 13 ihren Ort, ohne daß damit der Abstand zwischen den beiden Lagen verändert wird.

Aus der Dàrstellung gemäß Fig. 4, also einem Schnitt um 90°
gegenüber Fig. 3 sind die hindernisfreien Dialysatwege 14
sehr gut erkennbar. Die Fließrichtung 2 des Dialysats erstreckt sich hier senkrecht zur Zeichenebene. Man sieht, wie
die Kettfäden 1 benachbarter Membranhalterungen unregelmässig gegeneinander versetzt  erscheinen und sich der Strömungspfad 9 jeweils quer in Richtung der Pfeile 7 erstreckt.

# BIBRACH & REHBERG

ANWALTSSOZIETÄT

BIBRACH & REHBERG, POSTFACH 738, D-3400 GÖTTINGEN

PATENTANWALT DIPL.-ING. RUDOLF BIBRACH
PATENTANWALT DIPL.-ING. ELMAR REHBERG

EUROPEAN PATENT ATTORNEYS
MANDATAIRES EN BREVETS EUROPÉENS

TELEFON: (0551) 45034/35

TELEX: 96616 bipat d

POSTSCHECKKONTO: HANNOVER
(BLZ 25010030) NR. 115763-301

BANKKONTEN: DEUTSCHE BANK AG GÖTTINGEN
(BLZ 26070072) NR. 01/85900
COMMERZBANK GÖTTINGEN
(BLZ 26040030) NR. 6425722

0053392

| IHR ZEICHEN<br>YOUR REF.<br>VOTRE REF. | IHR SCHREIBEN VOM<br>YOUR LETTER<br>VOTRE LETTRE | UNSER ZEICHEN<br>OUR REF.<br>NOTRE REF.<br>10.716a/AS5 | D-3400 GÖTTINGEN,<br>PÜTTERWEG 6<br><br>30.11.1981 |

SECON Gesellschaft für Separations- und Concentrationstechnik mbH, Wagenstieg 5, 3400 Göttingen

Dialysator aus aufeinandergestapelten semipermeablen
Schlauchabschnitten

Patentansprüche :

1. Dialysator mit einem Gehäuse und daran angeordneten Zu-
und Ableitungen für Blut und Dialysat und mit in dem Gehäuse
angeordneten, aufeinandergestapelten semipermeablen Schlauchabschnitten, die jeweils in ihrem Innern eine Membranhalterung aus ungewebtem Kunststoffmaterial sich kreuzender Fäden
aufweisen, die in den Schlauchabschnitten den Strömungspfad
für das Dialysat und zwischen den Schlauchabschnitten den dazu kreuzweise geführten Strömungspfad für das Blut bildet,
dadurch gekennzeichnet, daß die Membranhalterung in Fließrichtung (2) des Dialysates durch den Einsatz vergleichsweise
dicker Kettfäden (1) auf Abstand (5) eine Vielzahl hindernisfreier Dialysatwege (14) aufweist, und daß die Membranhalterung in Fließrichtung (7) des Blutes durch den Einsatz vergleichsweise dünner Schußfäden (3) auf Abstand (6) eine Vielzahl dünner Strömungspfade (9) für das Blut besitzt, und daß
die Schußfäden (3) auf den Kettfäden (1) stabilisiert sind.

2. Dialysator nach Anspruch 1, dadurch gekennzeichnet, daß
die Kett- und Schußfäden (1, 3) mit solchen geometrischen
Abmessungen vorgesehen sind, daß die Schlauchabschnitte möglichst wenig durchhängen und so ein Strömungspfad (9) von
einer mittleren Dicke von etwa 50 ° gebildet wird.

3. Dialysator nach einem der Ansprüche 1 oder 2, dadurch gekennzeichnet, daß die Kettfäden (1) im wesentlichen die Stabilität der Membranhalterung bestimmen, und daß die Stabilisierung der Schußfäden (3) auf den Kettfäden (1) durch eine
Verschweißung und/oder eine Schlingenbildung unter Einsatz
eines dünnen Schlingfadens (4) bewirkt ist.

4. Dialysator nach Anspruch 1, dadurch gekennzeichnet, daß
die die hindernisfreien Dialysatwege (14) bildenden Kettfäden (1) einen Durchmesser aufweisen, der einerseits für eine
Abfuhr abgeschiedenen Gases ausreichend groß und andererseits
für die Bildung dünner Dialysatfilme hinreichend klein ist.

5. Dialysator nach einem der Ansprüche 1 bis 4, dadurch gekennzeichnet, daß die Kettfäden (1) einen Durchmesser zwischen 0, 15 bis 0, 3 mm - vorzugsweise 0, 2 mm -, einen gegenseitigen Abstand (5) zwischen 0, 5 bis 1,0 mm - vorzugsweise
0, 6 mm -, die Schußfäden (3) einen Durchmesser von 0, 08 bis
0, 15 mm - vorzugsweise 0, 12 mm - und einen gegenseitigen
Abstand (6) von 1, 0 bis 2, 0 mm - vorzugsweise 1, 2 bis
1, 6 mm - aufweisen.

6. Dialysator nach einem der Ansprüche 1 bis 4, dadurch gekennzeichnet, daß bei Stabilisierung des Schußfadens (3) auf dem Kettfaden (1) durch einen Schlingfaden (4) dieser einen Durchmesser von etwa 0, 08 mm aufweist.

7. Dialysator nach einem der Ansprüche 1 bis 6, dadurch gekennzeichnet, daß der Kettfaden (1) aus Polyäthylen, der Schußfaden (3) aus Polyester oder Polyacryl und der Schlingfaden (4) aus Polyamid besteht.

8. Dialysator nach einem der Ansprüche 1 bis 7, dadurch gekennzeichnet, daß auf einzelnen abständig angeordneten Kettfäden (1) je zwei Schußfäden (3) unmittelbar nebeneinander und diese Schußfadenpaare dann wieder abständig zueinander stabilisiert sind.

9. Dialysator nach einem der Ansprüche 1 bis 8, dadurch gekennzeichnet, daß bei Einsatz eines Schlingfadens (4) mindestens zwei verschiedene Arten von Membranhalterungen abwechselnd im Stapel angeordnet sind, die sich dadurch unterscheiden, daß sie jeweils verschiedenen Abstand (6, 6´) benachbarter Schußfäden (3) aufweisen.

10. Dialysator nach Anspruch 9, dadurch gekennzeichnet, daß die beiden Abstände (6, 6´) auf einem gemeinsamen Teilungsraster aufgebaut sind und jeweils ein unterschiedliches ganzteiliges Vielfaches des Teilungsrasters ausmachen.

Fig.1

Fig.2

Fig.3

Fig.4